**Europäisches Patentamt**

**European Patent Office**

**Office européen des brevets**

(11) Veröffentlichungsnummer : **0 330 606 B1**

(12) **EUROPÄISCHE PATENTSCHRIFT**

(19)

(45) Veröffentlichungstag der Patentschrift :
**15.04.92 Patentblatt 92/16**

(51) Int. Cl.⁵ : **A61F 2/28**

(21) Anmeldenummer : **89810029.2**

(22) Anmeldetag : **12.01.89**

(54) **Knochenimplantat.**

(30) Priorität : **26.02.88 CH 719/88**

(43) Veröffentlichungstag der Anmeldung :
**30.08.89 Patentblatt 89/35**

(45) Bekanntmachung des Hinweises auf die
Patenterteilung :
**15.04.92 Patentblatt 92/16**

(84) Benannte Vertragsstaaten :
**AT BE DE ES FR GB IT NL SE**

(56) Entgegenhaltungen :
**EP-A- 0 189 546**
**DE-U- 8 600 970**
**FR-A- 2 548 533**
**US-A- 4 660 755**

(73) Patentinhaber : **GEBRÜDER SULZER
AKTIENGESELLSCHAFT
Zürcherstrasse 9
CH-8401 Winterthur (CH)**

(72) Erfinder : **Koch, Rudolf
Oberdorfstrasse 229
CH-8267 Berlingen (CH)**
Erfinder : **Heisig, Eduard
Lände 7
W-7764 Wangen/See (DE)**

EP 0 330 606 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Knochenimplantat mit einem metallenen Grundkörper als Träger für ein mehrlagiges, geflochtenes und bleibend verformbares Drahtnetz, dessen Lagen untereinander und mit dem Grundkörper durch örtliche Schweissstellen verbunden sind.

Die US-Patentschrift 4,660,755 zeigt ein Verfahren bei dem an den gegenüberliegenden Seiten eines Implantats Kissen aus Metalldraht über ihre ganze Flächenausdehnung von Elektroden zusammengepresst werden, damit an Berührungspunkten zwischen den Drähten und an deren Berührungspunkten zum Grundkörper Widerstandsschweissungen entstehen. Simultane über grosse Flächen gestreute Mikroverschweissungen, die mit einer Elektrode ausgeführt werden, beinhalten erhebliche Fertigungsrisiken. Die Streuung der Vorpressung zwischen den Drähten und zwischen Drähten und Grundkörper geht hier unmittelbar in die Qualität und Lage der Schweissstellen ein.

Zum anderen hat es sich bei Anordnungen wie in EP-A-189 546 gezeigt, dass die Fixierung der Lagen des Gitters untereinander und auf dem Grundkörper des Implantates durch Punktschweissungen an einzelnen Haftstellen aufwendig und mühsam ist. Aufgabe der Erfindung ist es daher das Aufbringen und Fixieren des Drahtnetzes durch Punktschweissungen zu vereinfachen. Dieses Ziel wird gemäss der vorliegenden Erfindung dadurch erreicht, dass der Grundkörper an den vorgesehenen Schweissstellen aus einer Auflagebasis für das Drahtnetz stufenförmig hervorstehende Erhöhungen hat.

Mit den Erhöhungen in der Auflagebasis für das Drahtnetz ergibt sich zum Fixieren des Drahtnetzes durch Punktschweissungen ein Verfahren gemäss Anspruch 2, bei dem in einem Arbeitsgang die Lagen des Drahtnetzes zunächst durch einen Stempel gegen die Auflagebasis des Grundkörpers als Amboss gepresst und dabei verdichtet werden und anschliessend an den Stellen der hervorstehenden Erhöhungen durch das Drahtnetz hindurch Schweissverbindungen zum Grundkörper hergestellt werden.

Beim Aufpressen des Drahtnetzes passen sich die Lagen durch bleibendes Verformen an die Oberflächengestalt des Auflagebasis des Grundkörpers an. Während im Bereich der Auflagebasis im allgemeinen, wenn überhaupt, nur eine leichte Kompression der Netzstruktur stattfindet, kommt es im Bereich der Erhöhungen zu erheblichen Formänderungen und Verdichtungen der einzelnen Lagen des Netzes. Dadurch entsteht an diesen Stellen eine relativ grosse Berührungsfläche zwischen dem Grundkörper und dem Drahtnetz sowie zwischen dessen einzelnen Lagen.

An diesen Stellen finden dann Punktschweisselektroden, die mit Vorteil bereits in dem Stempel für das Verdichten des Drahtnetzes angeordnet sind, eine ausreichende metallische Brücke, entlang der ein "Strang" flüssigen Metalls entsteht, der zu einer Schweissnaht erstarrt.

Im folgenden wird die Erfindung anhand eines Ausführungsbeispiels im Zusammenhang mit der Zeichnung näher erläutert.

Fig. 1 zeigt schematisch in einem Schnitt ein zwischen einem Stempel und dem Grundkörper als Amboss gelagertes Drahtnetz vor dem Aufpressen auf den Grundkörper;

Fig. 2 gibt in gleicher Darstellung einen Grundkörper mit aufgepresstem Drahtnetz wieder, bei dem einzelne Haftstellen bereits verschweisst sind.

Das auf einem Grundkörper 1 zu befestigende Drahtnetz 2 besteht aus mehreren Lagen, bei denen die Maschenweite der Netzstruktur unterschiedlich ist und von aussen nach innen kleiner wird.

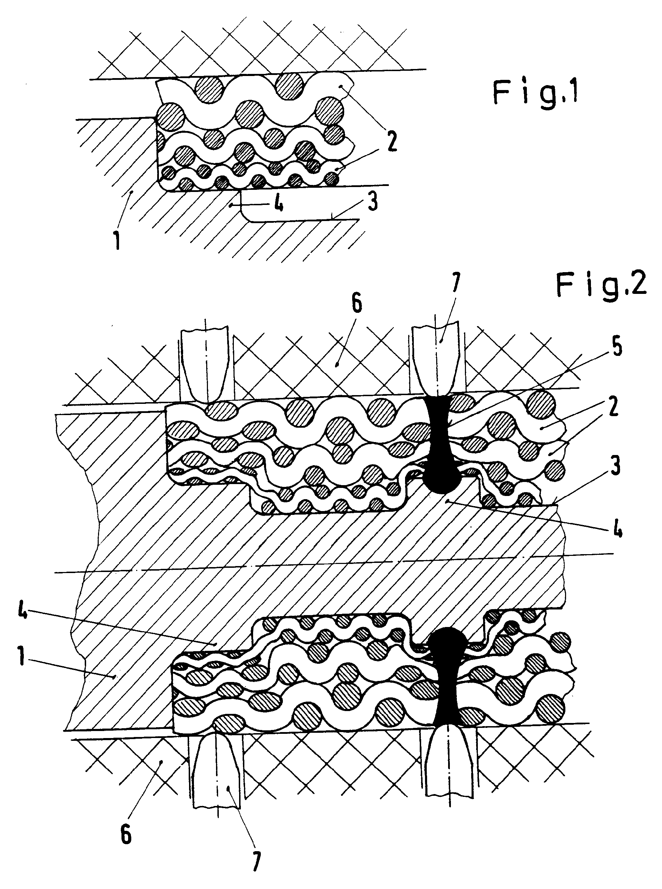

Für die Aufnahme des Drahtnetzes 2 hat der Grundkörper 1 einen stufenförmigen Querschnitt, dessen tiefstes Niveau die Auflagebasis 3 für das Netz 2 bildet. Erfindungsgemäss erheben sich aus dieser Basis 3 Erhöhungen 4; diese befinden sich an den Stellen, an denen die Lagen des Netzes miteinander und mit dem Grundkörper 1 durch Punktschweissungen 5 (Fig. 2) verbunden werden.

Das Einpressen des Netzes 2 in die Oberfläche des Grundkörpers 1 erfolgt mit Hilfe eines Stempels 6, in den Schweisselektroden 7 (Fig. 2) eingesetzt sind.

Die Lage der Erhöhungen 4 und der Elektroden 7 sind selbstverständlich zueinander koordiniert, damit die Schweissungen für die Punkte 5 auf den Erhöhungen 4 erfolgen.

Wie aus Fig. 2 zu entnehmen ist, sind die Lagen des Drahtnetzes 2 nach dem Einpressen in den Grundkörper 1 im Bereich der Erhöhungen 4 stark verdichtet und bleibend verformt, während in dem dazwischen liegenden tieferen Bereich der Auflagebasis 3 allenfalls leichte Kompressionen ohne bleibende Verformungen eintreten. Damit wird gewährleistet, dass auf der einen Seite genügend verdichtetes Material für die Ausbildung eines Schweisspunktes 5 vorhanden ist und auf der anderen Seite die Porosität des Drahtnetzes 2, die für die Aufnahme von Knochenzement oder das Einwachsen von Knochengewebe dienen soll, nicht unzulässig beeinträchtigt wird.

EP 0 330 606 B1

## Patentansprüche

1. Knochenimplantat mit einem metallenen Grundkörper als Träger für ein mehrlagiges, geflochtenes und bleibend verformbares Drahtnetz, dessen Lagen untereinander und mit dem Grundkörper durch örtliche Schweissstellen verbunden sind, **dadurch gekennzeichnet**, dass der Grundkörper (1) an den vorgesehenen Schweissstellen (5) aus einer Auflagebasis (3) für das Drahtnetz (2) stufenförmig hervorstehende Erhöhungen (4) hat.

2. Verfahren zum Fixieren der Lagen des Drahtnetzes untereinander und auf dem metallenen Grundkörper (1) eines Knochenimplantats nach Anspruch 1, wobei in einem Arbeitsgang die Lagen des Drahtnetzes (2) zunächst durch einen Stempel (6), in welchem Punktschweisselektroden (7) angebracht sind, gegen die Auflagebasis des Grundkörpers (1) als Amboss gepresst und dabei verdichtet werden und anschliessend punktförmige Schweissverbindungen (5) durch das Drahtnetz (2) hindurch zum Grundkörper (1) hergestellt werden, indem durch entsprechende Anordnung der Punktschweisselektroden (7) die Punktschweissverbindungen (5) an den Stellen der hervorstehenden Erhöhungen (4) des Grundkörpers hergestellt werden.

## Claims

1. A bone implant having a metal main body as a support for a multilayer braided and permanently deformable wire mesh whose layers are connected to one another and to the main body by local welds, characterised in that the main body (1) has at the places (5) intended for the welds protuberances (4) which project stepwise from a foundation base (3) for the wire mesh (2).

2. A method of fixing the layers of the wire mesh to one another and to the metal main member (1) of a bone implant according to claim 1 wherein in a single working step the layers of the wire mesh (2) are first pressed by a ram (6), which has spot welding electrodes (7), against the foundation base of the main body (1) as anvil and compresed, whereafter spot weld connections (5) are made through the wire mesh (2) to the main body (1) by the spot weld connections (5) being made, as a result of an appropriate arrangement of the spot welding electrode (7), at the places of the projecting protuberances (4) of the main body.

## Revendications

1. Implant pour os ayant une âme métallique servant de support à un treillis métallique tissé multicouche, déformable de façon permanente, dont les couches sont reliées entre elles ainsi qu'à l'âme par l'intermédiaire de points de soudure locaux, caractérisé en ce que l'âme (1) possède, au niveau des endroits prévus pour les points de soudure (5), des paliers proéminents (4) faisant saillie par rapport à une base d'appui (3) réceptrice du treillis métallique (2).

2. Procédé de fixation des couches du treillis métallique les unes sur les autres et sur l'âme métallique (1) d'un implant pour os selon la revendication 1, caractérisé en ce que l'on applique d'abord, sous pression, à l'aide d'un piston (6), dans lequel sont agencées des électrodes (7) de soudure par points, les couches du treillis métallique (2) sur la base d'appui de l'âme (1) qui sert d'enclume, compactant ainsi les différentes couches, et en ce que l'on réalise ensuite, au cours de la même opération, des points de soudure (5) traversant le treillis métallique (2), reliant ce dernier à l'âme (1), ces points de soudure (5) étant réalisés, grâce à une disposition appropriée des électrodes (7) de soudure par points, aux endroits correspondant aux paliers proéminents (4) de l'âme.

3

Fig.1

Fig.2